# EUROPÄISCHE PATENTSCHRIFT

(11) **EP 2 382 155 B1**
(45) Veröffentlichungstag und Bekanntmachung des Hinweises auf die Patenterteilung: **14.06.2017**
(21) Anmeldenummer: 09801448.3
(22) Anmeldetag: 22.12.2009
(51) Int. Cl.: C01B 17/16, C07C 319/02

(54) **VERFAHREN ZUR VERDICHTUNG VON SCHWEFELWASSERSTOFFHALTIGEN GASEN**
METHOD FOR COMPRESSING GASES CONTAINING HYDROGEN SULFIDE
PROCÉDÉ DE COMPRESSION DE GAZ CONTENANT DU SULFURE D'HYDROGÈNE

(30) Priorität: 23.12.2008 EP 08172787
(43) Veröffentlichungstag der Anmeldung: 02.11.2011
(73) Patentinhaber: BASF SE, 67056 Ludwigshafen am Rhein (DE)
(72) Erfinder: WÖLFERT, Andreas, 74906 Bad Rappenau (DE); GRZONKOWSKI, Frank, 67269 Grünstadt (DE); JACHOW, Harald, 64625 Bensheim (DE); RENZ, Günter, 67251 Freinsheim (DE)
(74) Vertreter: BASF IP Association
(86) Internationale Anmeldenummer: PCT/EP2009/067739
(87) Internationale Veröffentlichungsnummer: WO 2010/072756

(56) Entgegenhaltungen:
- EP-A1- 0 850 922
- WO-A1-00/31027
- DE-A1- 3 610 580
- DE-A1- 10 116 817
- DE-A1- 10 137 773
- DE-A1- 19 854 427
- US-A- 4 248 717

## Beschreibung

Die vorliegenden Erfindung betrifft ein Verfahren zur Verdichtung eines schwefelwasser-stoffhaltigen Gasstroms umfassend
(i) Verdichten des schwefelwasserstoffhaltigen Gasstroms in einem Verdichter,
(ii) Spülen des Verdichters mit einer Mischung enthaltend Dialkylpolysulfide, Dialkyldisulfide und mindestens ein Amin;
bevorzugt ein Verfahren zur Herstellung von Schwefelverbindungen ausgewählt aus der Gruppe bestehend aus Alkylmercaptanen, Dialkyldisulfiden und Alkansulfonsäuren umfassend die Umsetzung von schwefelwasserstoffhaltigen Gasströmen, wobei die schwefelwasserstoffhaltigen Gasströme gemäß dem Verfahren umfassend die Schritte i) und ii) verdichtet werden; sowie die Verwendung einer Mischung enthaltend Dialkyl-polysulfide, Dialkyldisulfide und mindestens ein Amin zur Entfernung von bei der Verdichtung von schwefelwasserstoffhaltigen Gasströmen auftretenden Schwefelablagerungen.

Die Verdichtung von schwefelwasserstoffhaltigen Gasströmen ist eine häufige verfahrenstechnische Fragestellung. Die Anwendung von Schwefelwasserstoff erfolgt häufig unter Druck. So wird zum Beispiel die Fällung von Schwermetallen aus Lösung unter Druck durchgeführt. Dadurch können Fällapparate mit geringem Volumen eingesetzt werden. Des Weiteren wird die Synthese von Alkylmercaptanen durch Umsetzung von Schwefelwasserstoff mit Alkanolen üblicherweise mittels verdichtetem Schwefelwasserstoff durchgeführt. Die Alkylmercaptanen können anschließend weiter umgesetzt werden, z.B. zu Dialkylsulfiden und/oder Alkansulfonsäuren.

Der in den vorstehend genannten Verfahren eingesetzte Schwefelwasserstoff kann zum Beispiel aus einer Sauergaswäsche, aus Raffinerieprozessen oder durch Umsetzung der Elemente Schwefel und Wasserstoff gewonnen werden. Wegen der Giftigkeit des Schwefelwasserstoffs ist man bemüht, sowohl die Menge an Schwefelwasserstoff, die in einem Produktionsprozess gehandhabt wird, als auch den Druck, unter dem der Schwefelwasserstoff gehandhabt wird, möglichst gering zu halten. Daher wird der Schwefelwasserstoff üblicherweise weitestgehend drucklos her- bzw. bereitgestellt und erst in der Anwendungsstufe verdichtet.

Bei der Verdichtung schwefelwasserstoffhaltiger Gasströme beobachtet man häufig, dass sich der Verdichter mit Schwefelablagerungen zusetzt. Diese können zu einem Abfall der Verdichterleistung oder, wenn der Verdichter trotz Ablagerungen weiterbetrieben wird, zu mechanischen Schäden am Verdichter führen.

Eine Ursache für die Schwefelablagerungen ist der Mitriss von elementarem Schwefel aus der eingesetzten schwefelwasserstoffhaltigen Quelle, der sich insbesondere an kalten Oberflächen ablagert. Eine weitere Ursache ist die Zersetzung von in der schwefelwasserstoffhaltigen Quelle im Allgemeinen enthaltenen Polysulfanen (H₂Sₓ, insbesondere zu Schwefelwasserstoff und Schwefel).

Ein wesentlicher Faktor für die Verfügbarkeit von Produktionsanlagen zur Synthese von Alkylmercaptanen bzw. daraus hergestellten Dialkyldisulfiden und Sulfonsäuren ist somit die Verfügbarkeit des Verdichters für das schwefelwasserstoffhaltige Gas.

Eine Möglichkeit, die Verfügbarkeit des Verdichters zu erhöhen, ist die Reinigung von schwefelwasserstoffhaltigen Gasen vor einer Einleitung in den Verdichter.

So betrifft WO 2004/022482 die Reinigung von Schwefelwasserstoff durch poröse Medien. Gemäß WO 2004/022482 wird durch Umsetzung von Wasserstoff und flüssigem Schwefel erhaltenes schwefelwasserstoffhaltiges Gas zur Reinigung durch einen Filter geleitet, der einen Feststoff ausgewählt aus Aktivkohle, Aluminiumoxid und Siliziumdioxid enthält. Gemäß WO 2004/022482 ist das so gereinigte Gas lediglich in geringem Maß oder gar nicht zur Abscheidung von festem Schwefel befähigt.

DE 102 45 164 A1 betrifft ein Verfahren zur Umwandlung von Polysulfanen. Diese Po-lysulfane H₂Sₓ fallen bei der Synthese von Schwefelwasserstoff durch Umsetzung von Wasserstoff mit Schwefel an. Bei Verdichtung des schwefelwasserstoffhaltigen Gasstroms kommt es zu einer unkontrollierten Zersetzung der Polysulfane in Schwefelwasserstoff und Schwefel, was zu unerwünschten Schwefelablagerungen im gesamten Verdichtungsbereich führt. Gemäß DE 102 45 164 A1 werden die Polysulfane in dem schwefelwasserstoffhaltigen Gas aus der Synthese durch Umsetzung von Wasserstoff und Schwefel katalytisch in Schwefelwasserstoff und Schwefel umgewandelt, indem man das schwefelwasserstoffhaltige Gas mit katalytisch wirksamen Festkörpern, katalytisch wirksamen Flüssigkeiten oder Gasen in Kontakt bringt. Geeignete katalytisch wirksame Festkörper sind gemäß DE 102 45 164 A1 Aktivkohle, Al₂O₃, SiO₂, Zeolithe, Gläser, Oxide und Mischoxide, Alkali-, Erdalkali und andere basische Gemische oder Hydroxide. Geeignete katalytisch wirksame Flüssigkeiten sind basische, wässrige oder allkoholische Lösungen von Ammoniak, Aminen oder Aminoalkoholen sowie Lösungen von Alkali-, Erdalkali- oder anderen basischen Oxiden oder Hydroxiden, Sulfiden oder Hydrogensulfiden. Geeignete Gase sind Ammoniak, Amine oder Aminoalkohole.

Nachteilig an den vorstehend genannten Verfahren zur Reinigung des aus der Umsetzung von Wasserstoff mit Schwefel gewonnenen Schwefelwasserstoffs ist der andauernde Verbrauch der zur Reinigung eingesetzten Komponenten.

Alternativ wird vorgeschlagen, die schwefelhaltigen Verunreinigungen in schwefelwasserstoffhaltigen Gasen durch Auskondensieren oder Desublimieren in alternierend arbeitenden Wärmetauschern zu entfernen, die dann bei Bedarf durch Aufheizen von Schwefelablagerungen befreit werden können (siehe Ullmann's Encyclopedia of Industrial Chemistry, Release 2008, 7th Edition, DOI: 10.1002/14356007.a13_467, Kapitel "Hydrogen Sulfide", Abschnitt "4.1 Production by chemical reaction").

Grundsätzlich ist des Weiteren eine mechanische Reinigung des Verdichters von Schwefelwasserstoffablagerungen möglich. Dies ist jedoch immer mit einer Anlagen-öffnung und der potentiellen Emission von Schwefelwasserstoff verbunden. Außerdem ist die Anlagenöffnung bei Anlagen mit toxischen Medien wegen der vorausgehenden Reinigungsprozesse mit langen Abstellzeiten verbunden. EP 0 850 922 A1 offenbart ein Verfahren zum Herstellen eines verdichteten schwefelwasserstoffhaltigen Gasstromes, umfassend das Verdichten eines schwefelwasserstoffhaltigen Gasstromes in einem Verdichter.

Die Aufgabe der vorliegenden Erfindung ist daher die Bereitstellung eines Verfahrens zur Verdichtung von schwefelwasserstoffhaltigen Gasen mit möglichst hoher Verfügbarkeit in Bezug auf den Verdichter, das mit einem möglichst geringen Investitionsaufwand auskommt.

Diese Aufgabe wird gelöst durch ein Verfahren zur Verdichtung eines schwefelwasserstoffhaltigen Gasstroms umfassend
(i) Verdichten des schwefelwasserstoffhaltigen Gasstroms in einem Verdichter,
(ii) Spülen des Verdichters mit einer Mischung enthaltend
   a) 10 bis 98 Gew.-% Dialkylpolysulfide;
   b) 1,9 bis 80 Gew.-% Dialkyldisulfide und
   c) 0,1 bis 10 Gew.-% mindestens eines Amins
wobei die Gesamtsumme an Dialkylpolysulfiden, Dialkyldisulfiden und mindestens einem Amin 100 Gew.-% beträgt.

### Schritt i)

Unter "Verdichter" ist im Sinne der vorliegenden Anmeldung nicht nur der Verdichter selbst zu verstehen sondern auch dessen Peripherie, das heißt insbesondere der Verdichter und einer oder mehrere angeschlossene Wärmetauscher, z.B. Rohrbündel- oder Plattenwärmetauscher, sowie gegebenenfalls angeschlossene Flüssigkeitsab-scheider.

Als Verdichter sind in dem vorliegenden Verfahren grundsätzlich alle dem Fachmann bekannten Verdichter geeignet. Bevorzugt werden in dem Verfahren gemäß der vorliegenden Anmeldung Kreisgasverdichter, besonders bevorzugt Schraubenverdichter oder Flüssigkeitsringverdichter eingesetzt, wobei Flüssigkeitsringverdichter ganz besonders bevorzugt sind. Kreisgasverdichter, insbesondere Schraubenverdichter und Flüssigkeitsringverdichter sind dem Fachmann bekannt.

Der Schraubenverdichter gehört zu den rotierenden, zweiwelligen Verdrängerverdichtern mit innerer Verdichtung. Der Schraubenverdichter kann ein- oder zweistufig ausgeführt sein. Zur Kühlung des zu verdichtenden schwefelwasserstoffhaltigen Gasstroms kann während der Verdichtung ein Einspritzmedium eingedüst werden. Die Einspritzung kann vor der ersten und/oder zweiten Stufe erfolgen. Als Einspritzmedium können zum Beispiel Wasser oder ein Alkohol (zum Beispiel Methanol bei der Herstellung von Methylmercaptan) eingesetzt werden. Das Einspritzmedium verdampft teilweise oder vollständig während des Einspritz- und Verdichtungsprozesses und kühlt dadurch das Prozessgas. Nach der Verdichtung kann das Einspritzmedium auskondensiert werden oder im Prozessgas belassen werden (ein Beispiel für eine Verdichtung im Schraubenverdichter mit Methanol als Einspritzmedium ist zum Beispiel in DE-A 196 54 515 offenbart). Bei der Auskondensation des Einspritzmediums und/oder Kühlung kann dieses gegebenenfalls nach Filtration wieder als Einspritzmedium verwendet werden.

Bei dem besonders bevorzugt in dem erfindungsgemäßen Verfahren eingesetzten Flüssigkeitsringverdichter handelt es sich um einen rotierenden Verdrängerverdichter einwelliger Bauart. Als Ringflüssigkeit kann in dem gemäß dem erfindungsgemäßen Verfahren eingesetzten Flüssigkeitsringverdichter zum Beispiel Wasser oder die erfindungsgemäß zum Spülen des Verdichters eingesetzte Mischung enthaltend Dialkylpolysulfide, Dialkyldisulfide und mindestens ein Amin eingesetzt werden. In einer bevorzugten Ausführungsform wird Wasser als Ringflüssigkeit eingesetzt. Im Verdichtungsprozess mitgerissene Ringflüssigkeit wird nach Verlassen des Verdichters in einem Flüssigkeitsabscheider (Demistor, Trennapparat von Gas und Flüssigkeit) abgeschieden, durch einen Wärmetauscher zur Kühlung geleitet und von dort aus zurück in den Verdichter geführt. Geeignete Wärmetauscher sind zum Beispiel Rohrbündel oder Plattenwärmetauscher. Im Falle des Flüssigkeitsringverdichters treten die Schwefelablagerungen bei Verdichtung des schwefelwasserstoffhaltigen Gasstroms insbesondere im Flüssigkeitsringverdichter selbst sowie in dem bzw. den Wärmetauscher(n) auf.

Die Verdichtung kann in Schritt i) des erfindungsgemäßen Verfahrens einstufig, zweistufig oder mehrstufig erfolgen. Bevorzugt erfolgt die Verdichtung ein- oder zweistufig.

Ein Verdichter, der keine Schwefelablagerungen aufweist, weist im Allgemeinen eine so hohe Verdichterleistung auf, dass mehr Gas verdichtet wird, als für den nachfolgenden Schritt benötigt wird. Das überschüssige verdichtete Gas wird über einen Umgang um den Verdichter von der Druckseite auf die Saugseite des Verdichters zurückgeführt (und wieder dem Verdichter zugeführt). In dem Fall, wenn der Verdichter mit Schwefelablagerungen zugesetzt wird, nimmt die Verdichterleistung mit der Menge der Schwefelablagerungen im Verdichter ab. Das bedeutet, dass weniger Gas durch den Umgang zu dem Verdichter zurückgeführt wird. Wenn die Verdichterleistung aufgrund der Schwefelablagerungen so gering ist, dass kein Gas durch den Umgang mehr zurückgeführt wird, muss der Verdichter gespült werden.

### Betriebsbedingungen des Verdichters

Der Eintrittsdruck in den Verdichter beträgt im Allgemeinen 700 mbar bis 3000 mbar absolut, bevorzugt 1000 bis 2000 mbar absolut, besonders bevorzugt 1100 bis 1500 mbar absolut. Der Austrittsdruck aus dem Verdichter beträgt im Allgemeinen 1000 bis 7000 mbar absolut, bevorzugt 1500 bis 4000 mbar absolut, besonders bevorzugt 2000 bis 3200 mbar absolut, ganz besonders bevorzugt 2200 bis 2800 mbar absolut, wobei der Eintrittsdruck geringer ist als der Austrittsdruck. Die Druckbedingungen in dem in dem erfindungsgemäßen Verfahren eingesetzten Verdichter unterscheiden sich somit wesentlich von den Druckbedingungen in einem Bohrloch, worin üblicher-weise Drücke von etwa 80 bar herrschen.

Die Eintrittstemperatur in den Verdichter beträgt im Allgemeinen 10 bis 70°C, bevorzugt 15 bis 50°C, besonders bevorzugt 20 bis 30°C. Die Austrittstemperatur aus dem Verdichter beträgt im Allgemeinen 15 bis 200°C, bevorzugt 20 bis 100°C, besonders bevorzugt 25 bis 50°C, ganz besonders bevorzugt 30 bis 40°C. Im Allgemeinen ist die Eintrittstemperatur geringer als die Austrittstemperatur.

### Schwefelwasserstoffhaltiger Gasstrom

Der durch den Verdichter geleitete schwefelwasserstoffhaltige Gasstrom kann durch beliebige, dem Fachmann bekannte Verfahren hergestellt werden. Der Gasstrom kann entweder aus der Sauergaswäsche oder aus Raffinerieprozessen stammen oder aus den Elementen Schwefel und Wasserstoff gewonnen werden. Bevorzugt wird der schwefelwasserstoffhaltige Gasstrom aus den Elementen Schwefel und Wasserstoff in Anwesenheit eines Katalysators oder unkatalytisch hergestellt. Geeignete Verfahren zur Herstellung des schwefelwasserstoffhaltigen Gasstroms sind dem Fachmann bekannt.

Beispielsweise erfolgt die Herstellung eines schwefelwasserstoffhaltigen Gasstroms gemäß dem Stand der Technik durch das H₂S-Verfahren nach Girdler (Ullmann's Encyclopedia of Industrial Chemistry, 6th Edition, 2003, Vol. 17, Seite 291). Dabei wird Schwefelstoffwasserstoff unkatalytisch aus den Elementen Schwefel und Wasserstoff in einer Kolonne mit Einbauten und einem im Wesentlichen horizontal ausgerichteten, erweiterten Sumpf hergestellt. In den mit siedendem Schwefel gefüllten Sumpf wird Wasserstoff eingeleitet, welcher in die aufsteigende Gasphase strippt. Wasserstoff und aufsteigender Schwefel reagieren im Gasraum der Kolonne, wobei die dabei freiwerdende Reaktionswärme dem Produktgas durch Wäsche mit flüssigem Schwefel entzogen wird. Dazu wird aus dem Sumpf der Kolonne flüssiger Schwefel abgezogen, mit frischem kaltem Schwefel gemischt und am Kopf der Kolonne aufgegeben. Das Produktgas, das weitgehend Schwefelwasserstoff enthält, wird in zwei Wärmetauschern abgekühlt.

Eine katalytische Herstellung von Schwefelwasserstoff wird zum Beispiel in Angew. Chem., 74 Jahrgang 1962; Nr. 4; Seite 151 beschrieben. Dabei wird Wasserstoff durch ein von außen temperiertes Schwefelbad geleitet. Der mit Schwefeldampf beladene Wasserstoff tritt durch Bohrungen in einen Katalysatorraum ein. Nicht abreagierter Schwefel wird nach Verlassen des Katalysatorraums in einem oberen Teil des Schwefelwasserstoff-Auslassrohres kondensiert und gelangt über ein Rücklaufrohr in das Schwefelbad zurück. Der Katalysatorraum ist konzentrisch um das Schwefelwasser-stoff-Auslassrohr angeordnet.

Weitere Beispiele für eine katalytische Herstellung von Schwefelwasserstoff aus den Elementen Schwefel und Wasserstoff sind in DE 1 113 446 und US 2,863,725 beschrieben.

In DE 1 113 446 ist die katalytische Herstellung von Schwefelwasserstoff durch Umsetzung eines stöchiometrischen Gemisches von Wasserstoff und Schwefel an einem Kobalt- und Molybdänsalz auf einem Träger enthaltenden Katalysator bei Temperaturen unter 500°C, bevorzugt zwischen 300 und 400°C beschrieben. Der Katalysator ist hierbei in Rohren angeordnet, die von dem Gemisch von Wasserstoff und Schwefel durchströmt werden.

Gemäß US 2,863,725 wird Schwefelwasserstoff an einem Molybdän enthaltenen Katalysator aus Wasserstoff und Schwefel hergestellt, wobei gasförmiger Wasserstoff in einen eine Schwefelschmelze enthaltenden Reaktor eingeleitet wird und durch die Schwefelschmelze in Form von Gasblasen aufsteigt.

Die Herstellung des schwefelwasserstoffhaltigen Gasstroms wird üblicherweise bei Drücken von 0,7 bis 2 bar, bevorzugt 0,9 bis 1,5 bar, ganz besonders bevorzugt 1 bar bis 1,4 bar absolut durchgeführt.

Die Temperatur des nach der Herstellung erhaltenen schwefelwasserstoffhaltigen Gasstroms beträgt im Allgemeinen 10 bis 60°C, bevorzugt 15 bis 50°C, besonders bevorzugt 20 bis 45°C, ganz besonders bevorzugt 25 bis 40°C.

Bei den Schwefelwasserstoffsynthesen aus Wasserstoff und Schwefel finden sich in dem erhaltenen schwefelwasserstoffhaltigen Rohgasstrom in der Regel als Nebenprodukte Polysulfane (H₂Sₓ) in einer Menge von im Allgemeinen 10 bis 200 Gew.-ppm, bevorzugt 15 bis 100 Gew.-ppm, besonders bevorzugt 20 bis 75 Gew.-ppm, ganz besonders bevorzugt 25 bis 50 Gew.-ppm.

Die Polysulfane werden bevorzugt vor Durchführung des erfindungsgemäßen Verfahrens zur Verdichtung des schwefelwasserstoffhaltigen Gasstroms aus dem schwefelwasserstoffhaltigen Gasstrom entfernt. Dies kann zum Beispiel durch eines der in den vorstehend genannten Dokumenten WO 2004/022482 oder DE 102 45 164 A1 beschriebenen Verfahren erfolgen. Es ist ebenfalls möglich, die Polysulfane durch Durchleiten durch ein poröses Material (Aktivkohlefilter oder Molsiebe) zu reinigen, zum Beispiel wie in WO 2008/087125 offenbart.

Zusätzlich erfolgt in einer bevorzugten Ausführungsform eine weitere Vorreinigung des schwefelwasserstoffhaltigen Gasstroms durch Desublimieren in einem Wärmetauscher. Das den Desublimator verlassende Gas hat bevorzugt einen molekularen Schwefelanteil von 0,001 bis 5 Gew.-ppm, besonders bevorzugt 0,005 bis 2 Gew.-ppm, ganz besonders bevorzugt 0,01 bis 1 Gew.-ppm.

Die Reinheit des in dem erfindungsgemäßen Verfahren zur Verdichtung eingesetzten Schwefelwasserstoffs (schwefelwasserstoffhaltiger Gasstrom) beträgt im Allgemeinen 90 bis 99,9 Vol.%, bevorzugt 95 bis 99,8 Vol.-%, besonders bevorzugt 98 bis 99,7 Vol.-% und ganz besonders bevorzugt 99 bis 99,6 Vol.-%.

### Schritt ii)

In Schritt ii) erfolgt erfindungsgemäß das Spülen des Verdichters mit einer Mischung enthaltend
a) 10 bis 98 Gew.-% Dialkylpolysulfide;
b) 1,9 bis 80 Gew.-% Dialkyldisulfide und
c) 0,1 bis 10 Gew.-% mindestens eines Amins,
wobei die Gesamtsumme an Dialkylpolysulfiden, Dialkyldisulfiden und mindestens einem Amin 100 Gew.-% beträgt.

Der Einsatz von Dialkylpolysulfiden als Lösungsmittel zur Auflösung von Schwefelablagerungen in Leitungen, die zum Transport von schwefelhaltigen Materialien dienen, insbesondere Ablagerungen bei der Förderung von Erdgas aus schwefelreichen Erdgasquellen, ist dem Fachmann zum Beispiel aus DE 36 10 580 A1 oder US 4 248 717 bekannt. In der DE 36 10 580 A1 ist ein Verfahren zum Auflösen von Schwefel mittels eines flüssigen Dialkylpolysulfids beschrieben, wobei das Lösungsmittel aus einem Dimethylpolysulfid-Gemisch besteht,
umfassend 1 bis 3 Gew.-% Dimethyldisulfid, 35 bis 45 Gew.-% CH₃SₓCH₃, wobei x einen Wert von 3 bis 5 hat, und als Rest homologe Polysulfide, wobei x einen Wert von 6 oder mehr und insbesondere 6 bis 8 hat. Zusätzlich kann das eingesetzte Dialkylpolysulfid 2 bis 10 Gew.-% eines Amins, Amids, Mercaptans und/oder Mercaptids enthalten. Das in DE 36 0 580 A1 offenbarte Lösungsmittel dient zur Auflösung von Schwefelabscheidungen, die in Leitungen, die zum Transport von schwefelhaltigen Materialien dienen, auftreten können. Dabei ist das Problem der Schwefelablagerung gemäß DE 36 10 580 A1 bei an schwefelreichen Erdgasquellen von besonderer Bedeutung, wobei die stark schwefelhaltigen Gase zu Schwefelablagerungen an den Innenwänden der Rohrleitungen führen. Gemäß DE 36 10 580 A1 weist Dimethyldisulfid (DMDS) ein beträchtliches Lösevermögen für Schwefel auf. Da es für eine kontinuierliche Reinigung notwendig ist, dass die Zusammensetzung des verwendeten Lösungsmittels weitgehend konstant bleibt, ist es erforderlich, das im Stand der Technik üblicherweise eingesetzte Dimethyldisulfid durch Abbau der während der Reinigung aufgenommenen höheren Polysulfide zu regenerieren. Gemäß DE 36 10 580 A1 wurde gefunden, dass als Alternative zu Dimethyldisulfid Dimethylpolysulfid-Gemische mit der speziellen, vorstehend genannten Zusammensetzung zur Entfernung von Schwefelablagerungen in Leitungen, die zum Transport von schwefelhaltigen Materialien dienen, eingesetzt werden können.

Während DE 36 10 580 A1 jedoch ein Verfahren zum Auflösen von Schwefel in Leitungen betrifft, die schwefelwasserstoffhaltige Gase aus an schwefelreichen Erdgasquellen führen, betrifft die vorliegenden Erfindung ein Verfahren zur Verdichtung von schwefelwasserstoffhaltigen Gasen, dass ein Spülen des Verdichters mit einer Mischung enthaltend Dialkylpolysulfide, Dialkyldisulfide und mindestens ein Amin, wie in Anspruch 1 beschrieben, umfasst. Die Temperatur- und Druckbedingungen, die in den Leitungen gemäß DE 36 10 580 A1 herrschen, sowie die eingesetzten schwefelwasserstoffhaltigen Gasströme unterscheiden sich beträchtlich von den bei der Verdichtung von schwefelwasserstoffhaltigen Gasen auftretenden Bedingungen und eingesetzten schwefelwasserstoffhaltigen Gasströmen. Des Weiteren sind auch die Vorrichtungen, auf denen die Schwefelablagerungen beobachtet werden, verschieden. Während DE 36 10 580 A1 die Problematik der Schwefelablagerung in Rohrleitungen betrifft, betrifft die vorliegende Anmeldung das Problem von Schwefelablagerungen in Verdichtern, wozu auch die Peripherie der Verdichter, zum Beispiel Wärmetauscher, gezählt wird. Die Verdichter und insbesondere die an die Verdichter angeschlossenen Wärmetauscher zeichnen sich dadurch aus, dass sie enge Spalten aufweisen. Ein Fachmann würde für die Reinigung der Spalten niedrigviskose Spüllösungen einsetzen.

Die Eignung von Mischungen, die Dialkylpolysulfide, Dialkyldisulfide und mindestens ein Amin enthalten, zum Spülen des Verdichters, ist daher bei Kenntnis von DE 36 10 580 A1 insbesondere aus den folgenden Gründen nicht nahe liegend:
In Bohrlöchern herrschen im Allgemeinen Drücke von etwa 80 bar, während der Verdichter im Allgemeinen bei einem Eintrittsdruck von 700 mbar bis 3000 mbar absolut und einem Austrittsdruck von 1000 bis 7000 mbar absolut betrieben wird.

Des Weiteren unterscheiden sich die strukturellen Gegebenheiten in Bohrlöchern, wobei es sich im Allgemeinen um Rohre von mehreren cm Durchmesser handelt, wesentlich von den strukturellen Gegebenheiten eines Verdichters und dessen Peripherie, der im Allgemeinen enge Spalten im mm-Bereich aufweist.

Diese Unterschiede führen dazu, ein Fachmann die hochviskosen Mischungen gemäß DE 36 10 580 A1, die zum Spülen von Bohrlöchern geeignet sind, nicht zum Spülen eines Verdichters und dessen Peripherie einsetzen würde, da diese hochviskosen Mischungen bei den im Verdichter herrschenden Druckbedingungen nur schlecht in die engen Spalten des Verdichters gelangen können und ebenso schlecht wieder herausgewaschen werden können.

Ein Fachmann würde zum Spülen des Verdichters daher viel eher die wesentlich niedriger viskosen Lösungen, enthaltend Dialkyldisulfide einsetzen, die üblicherweise zur Entfernung von Schwefelablagerungen in Bohrlöchern eingesetzt werden, wie aus der Publikation "Production challenges in developing sour gas reserves", Chemical Engineering World 24(3), 87-93, Hyne. J.B. hervorgeht, worin Dimethyldisulfid als bestes Lösungsmittel für Schwefel (Seite 91) bei der Anwendung in Sauergasbohrlöchern dargestellt wird.

Die üblicherweise zur Entfernung von Schwefelablagerungen in Bohrlöchern eingesetzten Dialkyldisulfide erweisen sich jedoch überraschenderweise als nicht gut geeignet zur schnellen und dauerhaften Entfernung von Schwefelablagerungen in einem Verdichter, wie die Vergleichsbeispiele in der vorliegenden Anmeldung zeigen. Überraschenderweise wurde gefunden, dass mit der erfindungsgemäß eingesetzten Mischung enthaltend Dialkylpolysulfide, Dialkyldisulfide und mindestens ein Amin, trotz der hohen Viskosität wesentlich bessere Ergebnisse zur schnellen und dauerhaften Entfernung von Schwefelablagerungen in einem Verdichter erzielt werden.

Des Weiteren haben die unterschiedlichen Druck- und Temperaturbedingungen in den Rohrleitungen gemäß DE 36 10 580 A1 und in dem Verdichter gemäß der vorliegenden Anmeldung zur Folge, dass die Schwefelablagerungen in den jeweiligen Vorrichtungen unterschiedliche Modifikationen haben können. Unterschiedliche Schwefelmodifikationen zeigen ein deutlich unterschiedliches Lösungsverhalten. Rhombischer Schwefel S₈ weist zum Beispiel eine wesentlich höhere Reaktivität und Löslichkeit auf als der so genannte µ-Schwefel. Es ist bekannt, dass eine schnelle Unterkühlung an kalten Flächen, zum Beispiel an Wärmetauscherflächen, zu µ-Schwefel führt. Die Art der Schwefelablagerung und damit die Löslichkeit der Schwefelablagerungen hängt wesentlich von der Temperaturgeschichte und der Herkunft des schwefelwasserstoffhaltigen Gases ab, aus dem die Schwefelablagerungen resultieren. Erfahrungen zur Beseitigung von Schwefelablagerungen aus einem heißen Bohrloch können somit nicht ohne weiteres auf die Beseitigung von Schwefelablagerungen in einem Verdichter übertragen werden. Es handelt sich um zwei vollständig unterschiedliche technische Gebiete.

Das Spülen des Verdichters gemäß Schritt ii) des erfindungsgemäßen Verfahrens kann kontinuierlich oder diskontinuierlich erfolgen.

Bei der kontinuierlichen Spülung wird der Verdichter kontinuierlich von den Schwefelablagerungen mit Hilfe der erfindungsgemäß eingesetzten Mischung enthaltend Dialkylpolysulfide, Dialkyldisulfide und mindestens ein Amin, wie in Anspruch 1 beschrieben, lefreit. Dabei wird die erfindungsgemäß eingesetzte Mischung z.B. bei Einsatz eines Flüssigkeitsringverdichters der Ringflüssigkeit zugegeben, die in den Verdichter zurückgeführt wird. Eine kontinuierliche Durchführung des erfindungsgemäßen Verfahrens bei Verwendung anderer Verdichter ist für einen Fachmann aufgrund seiner Kenntnisse problemlos möglich. Die Druck- und Temperaturbedingungen in dem Verdichter bei der kontinuierlichen Durchführung entsprechen den vorstehend genannten Druck- und Temperaturbedingungen im Verdichter.

Bei dem diskontinuierlichen Spülen wird der Verdichter kurzzeitig für Reinigungsarbeiten abgestellt und mit Hilfe der erfindungsgemäß eingesetzten Mischung enthaltend Dialkylpolysulfide, Dialkyldisulfide und mindestens ein Amin, wie in Anspruch 1 beschrieben, behandelt.

Üblicherweise erfolgt das diskontinuierliche Spülen des Verdichters mit der erfindungsgemäß eingesetzten Mischung enthaltend Dialkylpolysulfide, Dialkyldisulfide und mindestens ein Amin, wie in Anspruch 1 beschrieben, in der Form, dass der Verdichter von der Mischung in flüssiger Form durchströmt wird. Die Temperatur beträgt bei dem Spülvorgang im Allgemeinen 30 bis 160°C, bevorzugt 40 bis 140°C, besonders bevorzugt 60 bis 120°C, ganz besonders bevorzugt 75 bis 110°C und insbesondere ganz besonders bevorzugt 90 bis 100°C.

Bei einer diskontinuierlichen Spülung wird der Verdichter mit der zum Spülen eingesetzten Mischung teilweise oder vollständig gefüllt. Bevorzugt erfolgt die Füllung vollständig. Bei diskontinuierlicher Spülung betragen die Spülzeiten von 5 Minuten bis 1 Stunde, bevorzugt 10 Minuten bis 50 Minuten, besonders bevorzugt 20 Minuten bis 40 Minuten.

### Dialkyldisulfide und Dialkylpolysulfide

Geeignete Alkylgruppen der Dialkyldisulfide und Dialkylpolysulfide sind in jedem Dialkyldisulfid bzw. Dialkylpolysulfid unabhängig voneinander üblicherweise C₁-C₁₄-Alkylreste, bevorzugt C₁-C₆-Alkylreste, besonders bevorzugt C₁-C₃-Alkylreste. Ganz besonders bevorzugt handelt es sich bei den Alkylresten um Methyl, Ethyl, n-Propyl oder iso-Propyl, ganz besonders bevorzugt handelt es sich bei den Alkylresten um Methylreste. Die Alkylreste können in den Dialkylpolysulfiden bzw. Dialkyldisulfinden jeweils gleich oder verschieden sein. Bevorzugt sind sie gleich. Besonders bevorzugt handelt es sich bei den in dem erfindungsgemäßen Verfahren eingesetzten Dialkylpo-lysulfiden um Dimethylpolysulfide und bei den Dialkyldisulfiden um Dimethyldisulfid.

Geeignete Dialkylpolysulfide weisen die allgemeine Formel R-Sₓ-R' auf, wobei R und R' die vorstehend genannten Alkylreste sind. x bedeutet in den Dialkylpolysulfiden 3 bis 12, bevorzugt 3 bis 10. Üblicherweise liegen die Dialkylpolysulfide in Form von Mischungen von Dialklpolysulfiden mit verschiedenen Kettenlängen vor.

### Amine

Das in der erfindungsgemäß zum Spülen in Schritt ii) eingesetzten Mischung enthaltene mindestens eine Amin kann ein primäres, sekundäres oder tertiäres aliphatisches oder aromatisches Amin sein. Bevorzugt werden primäre, sekundäre oder tertiäre aliphatische Amine eingesetzt. Besonders bevorzugt sind flüssige oder feste Amine, die eine geringe Wasserlöslichkeit aufweisen. Ganz besonders bevorzugt handelt es sich bei den Aminen um primäre, sekundäre oder tertiäre Amine mit 6 bis 60 Kohlenstoffatomen. Beispiele für geeignete Amine sind Tridecylamin, Fettamine wie N,N-Dimethyl-C₁₂/C₁₄-Amin sowie Dicyclohexylamin.

Die erfindungsgemäß in Schritt ii) zum Spülen eingesetzten Mischungen enthaltend Dialkylpolysulfide, Dialkyldisulfide und mindestens ein Amin enthalten
a) 10 bis 98 Gew.-%, bevorzugt 20 bis 95 Gew.-%, besonders bevorzugt 35 bis 90 Gew.-% Dialkylpolysulfide;
b) 1,9 bis 80 Gew.-%, bevorzugt 4,8 bis 72 Gew.-%, besonders bevorzugt 9,5 bis 60 Gew.-% Dialkyldisulfide;
c) 0,1 bis 10 Gew.-%, bevorzugt 0,2 bis 8 Gew.-%, besonders bevorzugt 0,5 bis 5 Gew.-% mindestens eines Amins;
wobei die Gesamtsumme an Dialkylpolysulfiden, Dialkyldisulfiden und mindestens einem Amin 100 Gew.-% beträgt.

Bevorzugte Dialkylpolysulfide, Dialkyldisulfide und Amine sind vorstehend genannt.

In Abhängigkeit davon, ob das Spülen in Schritt ii) des erfindungsgemäßen Verfahrens zur Verdichtung kontinuierlich oder diskontinuierlich durchgeführt wird, kann die bevorzugt eingesetzte Zusammensetzung der Dialkylpolysulfide enthaltenden Mischung variieren.

In einer bevorzugten Ausführungsform von Schritt ii) des erfindungsgemäßen Verfahrens werden Mischungen eingesetzt, enthaltend:
a) 50 bis 98 Gew.-%, bevorzugt 70 bis 95 Gew.-%, besonders bevorzugt 80 bis 90 Gew.-% Dialkylpolysulfide;
b) 1,9 bis 40 Gew.-%, bevorzugt 4,8 bis 22 Gew.-%, besonders bevorzugt 9,5 bis 15 Gew.-% Dialkyldisulfide;
c) 0,1 bis 10 Gew.-%, bevorzugt 0,2 bis 8 Gew.-%, besonders bevorzugt 0,5 bis 5 Gew.-% mindestens eines Amins;
wobei die Gesamtsumme an Dialkylpolysulfiden, Dialkyldisulfiden und mindestens einem Amin 100 Gew.-% beträgt.

Die vorstehend genannte Mischung wird besonders bevorzugt bei diskontinuierlicher Durchführung von Schritt ii) des erfindungsgemäßen Verfahrens eingesetzt.

In einer weiteren bevorzugten Ausführungsform von Schritt ii) des erfindungsgemäßen Verfahrens werden Mischungen eingesetzt, enthaltend:
a) 10 bis 70 Gew.-%, bevorzugt 20 bis 60 Gew.-%, besonders bevorzugt 35 bis 50 Gew.-% Dialkylpolysulfide;
b) 29,9 bis 80 Gew.-%, bevorzugt 38,8 bis 72 Gew.-%, besonders bevorzugt 49,5 bis 60 Gew.-% Dialkyldisulfide;
c) 0,1 bis 10 Gew.-%, bevorzugt 0,2 bis 8 Gew.-%, besonders bevorzugt 0,5 bis 5 Gew.-% mindestens eines Amins;
wobei die Gesamtsumme an Dialkylpolysulfiden, Dialkyldisulfiden und mindestens einem Amin 100 Gew.-% beträgt.

Die vorstehend genannte Mischung wird besonders bevorzugt bei kontinuierlicher Durchführung von Schritt ii) des erfindungsgemäßen Verfahrens eingesetzt.

Die in Schritt ii) eingesetzte Mischung enthält keine weiteren Komponenten. Insbesondere enthalten die zum Spülen eingesetzten Mischungen in einer bevorzugten Ausführungsform der vorliegenden Erfindung keine Tenside.

In einer bevorzugten Ausführungsform der vorliegenden Erfindung handelt es sich bei den zum Spülen des Verdichters in Schritt ii) eingesetzten Mischungen nicht um Mischungen, die extra zum Zweck des Spülens hergestellt werden müssen, sondern um Mischungen, wie sie in Verfahren zur Herstellung von Schwefelverbindungen ausgehend von gemäß dem erfindungsgemäßen Verfahren verdichtetem schwefelwasserstoffhaltigem Gas anfallen. Solche Schwefelverbindungen sind zum Beispiel Alkylmercaptane, Dialkyldisulfide und Alkansulfonsäuren. Besonders bevorzugt handelt es sich bei den zum Spülen in Schritt ii) des erfindungsgemäßen Verfahrens eingesetzten Mischungen um den aminhaltigen Rohaustrag aus der Dialkyldisulfidsynthese, die gemäß dem Fachmann bekannten Verfahren durchgeführt werden kann und nachstehend näher erläutert wird.

Das erfindungsgemäße Verfahren zur Verdichtung eines schwefelwasserstoffhaltigen Gasstroms wird in einer Ausführungsform der vorliegenden Erfindung in Kombination mit der Herstellung von Schwefelverbindungen, insbesondere Schwefelverbindungen ausgewählt aus der Gruppe bestehend aus Alkylmercaptanen, Dialkyldisulfiden und Alkansulfonsäuren eingesetzt. Ein weiterer Gegenstand der vorliegenden Erfindung ist daher ein Verfahren zur Herstellung von Schwefelverbindungen ausgewählt aus der Gruppe bestehend aus Alkylmercaptenen, Dialkyldisulfiden und Alkansulfonsäuren umfassend die Umsetzung eines schwefelwasserstoffhaltigen Gasstroms, wobei der schwefelwasserstoffhaltige Gasstrom gemäß dem erfindungsgemäßen Verfahren zur Verdichtung des schwefelwasserstoffhaltigen Gasstroms umfassend die Schritt i) und ii) verdichtet wird.

### Verfahren zur Herstellung von Alkylmercaptanen

Verfahren zur Herstellung von Alkylmercaptanen sind dem Fachmann bekannt. Erfindungsgemäß wird zur Herstellung der Alkylmercaptane ein verdichteter schwefelwas-serstoffhaltiger Gasstrom eingesetzt, wobei die Verdichtung des zur Herstellung der Alkylmercaptane eingesetzten schwefelwasserstoffhaltigen Gasstroms die Schritte i) und ii) umfasst. Bevorzugt erfolgt die Herstellung der Alkylmercaptane durch Umsetzung von Alkanolen mit einem gemäß dem erfindungsgemäßen Verfahren umfassend die Schritte i) und ii) verdichteten schwefelwasserstoffhaltigen Gasstrom.

Die vorliegende Erfindung betrifft bevorzugt das erfindungsgemäße Verfahren zur Verdichtung eines schwefelwasserstoffhaltigen Gasstroms umfassend
(i) Verdichten des schwefelwasserstoffhaltigen Gasstroms in einem Verdichter,
(ii) Spülen des Verdichters mit einer Mischung enthaltend
   a) 10 bis 98 Gew.-% Dialkylpolysulfide;
   b) 1,9 bis 80 Gew.-% Dialkyldisulfide und
   c) 0,1 bis 10 Gew.-% mindestens eines Amins,
   wobei die Gesamtsumme an Dialkylpolysulfiden, Dialkyldisulfiden und mindestens einem Amin 100 Gew.-% beträgt
(iii) und anschließende Umsetzung des verdichteten schwefelwasserstoffhaltigen Gasstroms zu Alkylmercaptanen, Dialkyldisulfiden und Alkansulfonsäuren.

Die Schritte i) und ii) wurden vorstehend beschrieben.

### Schritt iii)

Die Umsetzung in Schritt iii) kann dabei nach üblichen, dem Fachmann bekannten Verfahren durchgeführt werden. Geeignete Verfahren sind zum Beispiel in DE 101 37 773 A1 genannt. Üblicherweise erfolgt die Umsetzung der Alkanole mit dem verdichteten schwefelwasserstoffhaltigen Gasstrom in Anwesenheit von Katalysatoren. Geeignete Katalysatoren sind zum Beispiel in US 2,874,129 (Metalloxide des Thoriums, Zirkons, Titans, Vanadium, Wolframs, Molybdäns oder Chroms auf einem porösen Träger, zum Beispiel Al₂O₃ oder Bimsstein); EP-A 0 749 961 (Alkalicarbonat auf Al₂O₃); EP-A 1 005 906 (Katalysator auf der Basis von Zirkonoxid, dotiert mit Magnesium oder Erdalkalimetallen), EP-A 0 038 540 (Zeolith-Katalysator mit reduzierter Menge an Alkalimetallkationen), EP-A 0 564 706 (Katalysator aus amorphem Al₂O₃-Gel bzw. aus auf einem aluminiumhaltigen Material aufgebrachtem Al₂O3-Gel); US 2,822,401 (aktiviertes Al₂O₃), US 2,820,062 (aktives Al₂O₃ mit 1,5 bis 15 Gew.-% Kaliumwolframat als Promotor), DE 196 39 584 (aktives Al₂O₃ mit 15 bis 40 Gew.-% Cäsiumwolframat als Promotor) und in US 5,874,630 (Al₂O₃ mit 0 bis 20 Gew.-% einer Übergangsmetallverbindung und 0,1 bis 10 Gew.-% eines Alkalimetall- oder Erdalkalimetallbicarbonats, -carbonats, -oxids- oder hydroxids) offenbart. Bevorzugt werden Katalysatoren auf der Basis von Al₂O₃, besonders bevorzugt γ-Al₂O₃, eingesetzt, das gegebenenfalls mit Promotoren dotiert ist. Geeignete Promotoren sind Übergangsmetallverbindungen ausgewählt aus der Gruppe bestehend WO₃, K₂WO₄, H₂WO₄, Cs₂WO₄, Na₂WO₄, MoO₃, K₂MoO₄, H₂MoO₄, Na₂MoO₄, Phosphowolframat, Phosphomolybdat und Silikowolframat. Der Anteil des Promotors beträgt im Allgemeinen 1 bis 40 Gew.-%, bevorzugt 5 bis 25 Gew.-%, bezogen auf das Gewicht des Katalysators. In der Alkylmercaptansynthese in Schritt iii) wird besonders bevorzugt als Katalalysator γ-Al₂O₃ eingesetzt, das mit K₂WO₄ als Promotor dotiert ist. Bevorzugte Alkylmercaptane weisen Alkylreste mit 1 bis 14 Kohlenstoffatomen, besonders bevorzugt 1 bis 6 Kohlenstoffatomen, ganz besonders bevorzugt 1 bis 3 Kohlenstoffatomen, das heißt Methyl-, Ethyl-, n-Propyl- oder iso-Propylreste, auf. Als Alkanole ROH werden somit in Schritt iii) des Verfahrens zur Herstellung von Alkylmercaptanen die entsprechenden Alkanole eingesetzt.

Im Allgemeinen wird Schritt iii) zur Herstellung von Alkylmercaptanen als Gasphasenreaktion durchgeführt. Dabei werden das Alkanol und der schwefelwasserstoffhaltige Gasstrom üblicherweise auf eine Temperatur geheizt, die hoch genug ist, dass sowohl das Alkanol als auch das gewünschte Alkylmercaptan in der Dampfphase vorliegen. Dabei darf die Temperatur nicht so hoch gewählt werden, dass eine Zersetzung des Alkylmercaptans eintritt. Im Allgemeinen wird das Verfahren gemäß Schritt iii) bei Temperaturen zwischen 250 und 500°C, bevorzugt zwischen 300 und 450°C durchgeführt. Der Druck beträgt im Allgemeinen 1 bis 25 bar, bevorzugt 1 bis 10 bar absolut.

Die erhaltenen Alkylmercaptane können direkt weiter zu Dialkyldisulfiden umgesetzt werden. Des Weiteren können die Alkylmercaptane zur Herstellung von Alkansulfonsäuren verwendet werden.

In einer bevorzugten Ausführungsform wird der mit Hilfe des vorstehend genannten Verfahrens hergestellte "Roh-Mercaptan-Strom", das heißt ein nicht durch Extraktion oder Destillation gereinigter Mercaptan-Strom, der nicht-abreagierten Schwefelwasserstoff, Wasser und als Nebenkomponenten Dialkylsulfid, geringe Mengen Alkanol und Dialkylether enthalten kann, zur Herstellung von Dialkyldisulfiden weiterverwendet.

### Herstellung von Dialkyldisulfiden

Die Dialkyldisulfide können nach beliebigen dem Fachmann bekannten Verfahren hergestellt werden, soweit sie einen Schritt zur Verdichtung von schwefelwasserstoffhaltigen Gasströmen umfassen. Die Verdichtung der schwefelwasserstoffhaltigen Gasströme erfolgt gemäß dem erfindungsgemäßen Verfahren umfassend die Schritte i) und ii).

In einer bevorzugten Ausführungsform werden die Dialkyldisulfide durch ein Verfahren hergestellt, umfassend
a) die Herstellung von Alkylmercaptanen umfassend die Schritte i), ii) und iii), die vorstehend erwähnt sind, und
b) die Umsetzung der in Schritt a) erhaltenen Alkylmercaptane durch Oxidation mit Schwefel zu Dialkyldisulfiden.

### Schritt a)

Die Herstellung der Alkymercaptane in Schritt a) erfolgt wie vorstehend bezüglich der Herstellung von Alkylmercaptanen beschrieben wurde.

### Schritt b)

Bei einer Herstellung der Dialkyldisulfide durch ein Verfahren umfassend die Schritte a) und b) erfolgt in Schritt b) die Umsetzung des in Schritt a) erhaltenen Alkymercaptans bevorzugt mit in einem organischen Disulfid gelöstem Schwefel unter Katalyse mit einem Amin. Geeignete Amine sind die vorstehend bezüglich der in der Mischung, die zum Spülen in Schritt ii) des erfindungsgemäßen Verfahrens eingesetzt wird genannten Amine.

Üblicherweise wird Schritt b) in einer Reaktionskolonne durchgeführt, wobei anfallende Leichtsieder in Schritt a) zurückgeführt werden.

In einer bevorzugten Ausführungsform schließt sich an Schritt b) des vorstehend genannten Verfahrens die Phasentrennung des erhaltenen Gemisches aus wässriger Phase, die ausgeschleust wird, und schwefelorganischer Phase an.

Anschließend erfolgt in einer weiteren bevorzugten Ausführungsform die Aufreinigung der schwefelorganischen Phase, die gegebenenfalls Leichtsieder, das gewünschte organische Disulfid, Polysulfide, Amin und geringe Mengen weiterer Nebenprodukte enthält, wobei das organische Disulfid entnommen wird, gegebenenfalls anfallende Leichtsieder in Schritt (a) zurückgeführt werden und anfallende Polysulfide und Amin in Schritt (b) zurückgeführt werden, unter Zusatz von Schwefel und gegebenenfalls Amin, wobei die Phasentrennung und das Ausschleusen der wässrigen Phase im Anschluss an Schritt (a) oder Schritt (b) erfolgen kann.

Bei dem als Lösungsmittel in Schritt (b) eingesetzten organischen Disulfid handelt es sich bevorzugt um das herzustellende organische Disulfid.

Ein besonders geeignetes Verfahren zur Herstellung der Dialkyldisulfide ist in DE 198 54 427 A1 genannt, worin in Schritt a) die Umsetzung von Alkanolen mit Schwefelwasserstoff an einem geeigneten Katalysator zu einem "Roh-Mercaptan-Strom" enthaltend Mercaptan, Wasser, Schwefelwasserstoff sowie geringe Mengen weiterer Nebenprodukte wie organisches Sulfid und Ether, erfolgt und in dem anschließenden Schritt b) die Umsetzung des "Roh-Mercaptan-Stroms" mit in einem organischen Disulfid gelöstem Schwefel unter Katalyse mit einem Amin. Erfindungsgemäß wird der in Schritt a) eingesetzte Schwefelwasserstoff gemäß dem Verfahren umfassend die Schritte i) und ii) verdichtet.

Ein weiteres bevorzugtes Verfahren zur Herstellung Dialkyldisulfiden ist in DE 101 16 817 A1 offenbart, worin die Herstellung von organischen Disulfiden in einer mit temperierbaren Böden ausgestatteten Kolonne aus einem Roh-Mercaptan-Strom ohne vorausgehende destillative Auftrennung erwähnt ist. Die Verdichtung des Schwefelwasserstoffs erfolgt erfindungsgemäß durch ein Verfahren umfassend die Schritte i) und ii), die vorstehend erwähnt sind.

### Herstellung von Alkansulfonsäuren

Ein weiterer Gegenstand der vorliegenden Erfindung betrifft ein Verfahren zur Herstellung von Alkansulfonsäuren, das ebenfalls ausgehend von gemäß dem erfindungsgemäßen Verfahren verdichteten schwefelwasserstoffhaltigen Gasströmen durchgeführt wird.

Geeignete Verfahren zur Herstellung von Alkansulfonsäuren ausgehend von verdichteten Schwefelwasserstoffgasströmen sind dem Fachmann bekannt.

In einer bevorzugten Ausführungsform werden die Alkansulfonsäuren durch ein Verfahren hergestellt umfassend
a) die Herstellung von Alkylmercaptanen durch Umsetzung mit Alkanolen mit einem gemäß dem erfindungsgemäßen Verdichtungsverfahren umfassend die Schritte i) und ii) verdichteten Gasstrom;
b) die Umsetzung der in Schritt a) hergestellten Mercaptane durch Oxidation mit Schwefel zu Dialkyldisulfiden; und
c) die Oxidation der in Schritt b) erhaltenen Dialkyldisulfide mit einem Oxidationsmittel zu Alkansulfonsäure.

Die Schritte a) und b) entsprechen dabei den bezüglich der Herstellung der Dialkyldisulfide erwähnten Schritten a) und b).

Die Oxidation in Schritt c) kann mit verschiedenen Oxidationsmitteln erreicht werden. So können als Oxidationsmittel Wasserstoffperoxid, Chlor, Dimethylsulfoxid, Gemische aus Dimethylsulfoxid und lodwasserstoffsäure sowie Salpetersäure oder Gemische der genannten Oxidationsmittel eingesetzt werden. Des Weiteren ist eine elektrochemische Oxidation möglich. Geeignete Verfahren zur Herstellung von Alkansulfonsäuren durch Oxidation der entsprechenden Dialkyldisulfide sind dem Fachmann bekannt und zum Beispiel in WO 98/34914, US 2,697,722, US 2,727,920 und WO 00/31027 offenbart.

Wesentlich bei den vorstehend genannten Verfahren zur Herstellung von Schwefelverbindungen ausgewählt aus Alkylmercaptanen, Dialkyldisulfiden und Alkansulfonsäuren ist, dass dieses Verfahren einen Schritt zur Verdichtung eines schwefelwasserstoffhaltigen Gasstroms umfassen, wobei die Verdichtung des schwefelwasserstoffhaltigen Gasstroms entsprechend dem erfindungsgemäßen Verfahren umfassend die Schritte i) und ii) durchgeführt wird.

In einer besonders bevorzugten Ausführungsform betrifft die vorliegende Erfindung ein Verfahren zur Herstellung von Dialkyldisulfiden, insbesondere ein Verfahren zur Herstellung von Dialkyldisulfiden, das die Schritte (a) und (b), wie sie vorstehend aufgeführt sind, umfasst.

Der Verdichter wird in dem erfindungsgemäßen Verfahren zur Herstellung von Dialkyldisulfiden bevorzugt mit einer Mischung enthaltend Dialkyldisulfide, Dialkylpolysulfide und mindestens ein Amin, wie in Anspruch 1 beschrieben, gespült, wobei diese Mischung der aminhaltige Rohaustrag aus der Dialkyldisulfidsynthese ist. Dabei sind bevorzugte Zusammensetzungen des Rohaustrags jeweils für ein diskontinuierliches Spülen bzw. ein kontinuierliches Spülen vorstehend genannt. Die zum Spülen eingesetzte Mischung kann nach dem Spülen in Schritt ii) des erfindungsgemäßen Verfahrens wieder in das Verfahren zur Herstellung von Dialkyldisulfiden, im Allgemeinen in den Sumpf der Dialkyldisulfid-Reindestillation (bevorzugt im diskontinuierlichen Spül-Verfahren in Schritt ii)) oder in den Sumpf der Dialkyldisulfid-Reaktionskolonne nach Abtrennung der wässrigen Phase (bevorzugt im kontinuierlichen Spül-Verfahren in Schritt ii)), zurückgeführt werden.

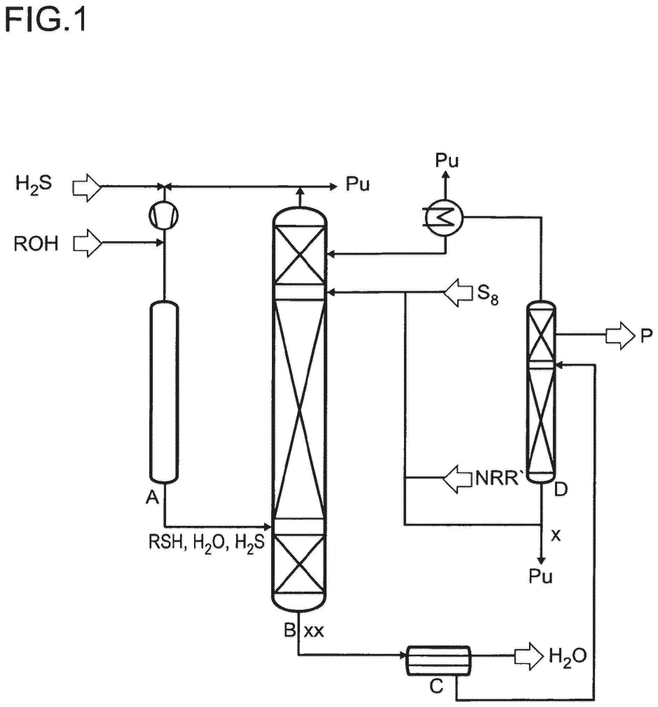

In Figur 1 ist ein Verfahrensschema dargestellt, worin die Herstellung von Dialkyldisulfiden gezeigt ist. Darin bedeuten:
- H₂S: Zuführung des schwefelwasserstoffhaltigen Gasstroms
- ROH: Zuführung von Alkanol
- V: Verdichter
- A: Reaktor zur Herstellung von Alkylmercaptan
- B: Reaktor zur Herstellung von Dialkyldisulfid, üblicherweise Dialkyldisulfid-Reaktionskolonne
- S₈: Zuführung von elementarem Schwefel
- NRR': Zuführung von Amin
- C: Phasenabscheider
- D: Dialkyldisulfid-Destillationskolonne, üblicherweise Dialkyldisulfid-Reindestillationskolonne
- H₂O: Entnahme der wässrigen Phase
- P: Produktentnahme (reines Dialkyldisulfid)
- Pu: Ausschleusestrom zur Vermeidung von Aufpegelungen (Purge)

Die Zeichen x bzw. xx zeigen, an welchen Stellen der aminhaltige Rohaustrag zum Spülen in Schritt ii) des erfindungsgemäßen Verdichtungsverfahrens bevorzugt entnommen wird. Dabei gibt die mit x gekennzeichnete Stelle den Entnahmepunkt aus dem Sumpf der Dialkyldisulfid-Reindestillation wider, d.h. der Mischung, die bevorzugt zum diskontinuierlichen Spülen eingesetzt wird und die mit xx gekennzeichnete Stelle den Entnahmepunkt aus dem Sumpf der Dialkyldisulfid-Reaktionskolonne nach Abtrennung der wässrigen Phase wider, d.h. der Mischung, die bevorzugt zum kontinuierlichen Spülen eingesetzt wird.

Aus dem Verfahrensschema in Figur 1 wird deutlich, dass die bevorzugt zum diskontinuierlichen Spülen in Schritt i) des Verdichtungsverfahrens eingesetzte Mischung bevorzugt dem Sumpf aus der Dialkyldisulfid-Reindestillation aus der letzten Kolonne D der Dialkyldisulfidsynthese entspricht. Die bevorzugt zum kontinuierlichen Spülen in Schritt ii) des erfindungsgemäßen Verdichtungsverfahrens eingesetzte Mischung entspricht bevorzugt dem Sumpf aus der Kolonne B nach Phasentrennung im Phasenscheider C, wobei die wässrige Phase abgetrennt wird und die organische Phase zum Spülen eingesetzt wird.

Ein wesentlicher Vorteil des erfindungsgemäßen Verfahrens ist es, dass die zum Spülen des Verdichters eingesetzte Mischung nicht aufwändig hergestellt werden muss, sondern dem aminhaltigen Rohaustrag aus der Dialkyldisulfidsynthese entspricht. Dies stellt insbesondere dann einen Vorteil dar, wenn der verdichtete schwefelwasserstoffhaltige Gasstrom zur Herstellung von Dialkyldisulfiden eingesetzt wird.

Das bei der Herstellung der vorstehend genannten Schwefelverbindungen durch den Verdichter geleitete schwefelwasserstoffhaltige Gas entspricht im Allgemeinen nicht dem zu Beginn des Verfahrens eingesetzten reinen schwefelwasserstoffhaltigen Gasstrom sondern ist ein im Kreis geführtes Gas, das üblicherweise 60 bis 90 Gew.-%, bevorzugt 70 bis 80 Gew.-% Schwefelwasserstoff, 2 bis 20 Gew.-%, bevorzugt 5 bis 15 Gew.-% Dimethylsulfid sowie geringe Teile Kohlenmonoxid, Methylmercaptan und Dimethylether enthält.

Ein weiterer Gegenstand der vorliegenden Erfindung betrifft die Verwendung einer Mi-schung enthaltend a) 10 bis 98 Gew.-% Dialkylpolysulfide; b) 1,9 bis 80 Gew.-% Dialkyldisulfide und c) 0,1 bis 10 Gew.-% mindestens eines Amins enthält, wobei die Gesamtsumme an Dialkylpolysulfiden, Dialkyldisulfiden und mindestens einem Amin 100 Gew.-% beträgt, zur Entfernung von bei der Verdichtung von schwefelwasserstoffhaltigen Gasströmen auftretenden Schwefelablagerungen. Diese Schwefelablagerungen treten dabei im Allgemeinen im Verdichter auf, wobei unter Verdichter sowohl der Verdichter selbst als auch dessen Peripherie - wie vorstehend ausgeführt - verstanden wird. Bevorzugt eingesetzte Mischungen sowie bevorzugt durchgeführte Verdichtungsverfahren sind vorstehend genannt.

Die nachfolgenden Beispiele erläutern die Erfindung zusätzlich.

### Beispiele

Die Beispiele werden jeweils mit einem Flüssigkeitsringverdichter mit Plattenwärmetauscher zur Kühlung der Ringflüssigkeit und Umgangsmöglichkeit durchgeführt, der aus durchgeführten Verdichtungsvorgängen von H₂S, das aus der Synthese aus den Elementen stammt, und in einem Verfahren zur Synthese von Methylmercaptan eingesetzt wird, Schwefelablagerungen aufweist. Die Schwefelablagerungen sind in den Beispielen und Vergleichsbeispielen identisch.

### 1. Vergleichsbeispiel - diskontinuierliche Spülung

Zur diskontinuierlichen Spülung wird der Verdichter und dessen Peripherie abgestellt und mit Dimethyldisulfid, das als gutes Lösungsmittel für Schwefel bekannt ist, gefüllt. Der Verdichter ist nach einer Einwirkzeit von 30 Minuten bei einer Temperatur von 90°C frei für einen weiteren Betrieb von 3 Wochen.

### 2. Erfindungsgemäßes Beispiel - diskontinuierliche Spülung

Wie in Beispiel 1 wird der Verdichter und dessen Peripherie abgestellt. Anstelle von Dimethyldisulfid wird der Verdichter jedoch mit einer Polysulfid-Lösung aus dem Sumpf der Dimethyldisulfid-Reindestillation gefüllt. Dazu werden der Verdichter und dessen Peripherie mit dem Sumpfaustrag gefüllt. Nach einer Einwirkzeit von 30 Minuten bei einer Temperatur von 90°C sind Verdichter und dessen Peripherie für einen weiteren Betrieb der Anlage von 3 Monaten frei. Die Spülflüssigkeit wird in den Sumpf der Di-methyldisulfid-Reindestillation zurückgeführt.

### 3. Erfindungsgemäßes Beispiel - kontinuierliche Spülung

Zum kontinuierlichen Spülen wird die organische Phase aus dem Phasenscheider nach der Dimethyldisulfid-Rohkolonne verwendet. Dazu werden dem Phasenscheider 0,5 bis 10 kg, bevorzugt 1 bis 5 kg organische Phase pro 100 kg zu verdichtendem Schwefelwasserstoff entnommen und in den Verdichter auf der Saugseite bzw. der Ringflüssigkeitszufuhr bei Verwendung eines Flüssigkeitsringverdichters zugeführt. Die nach dem Verdichter abgetrennte Flüssigphase wird bevorzugt in den Sumpf der Dimethyldisulfid-Reaktionskolonne zurückgeführt.

## Patentansprüche

1. Verfahren zur Verdichtung eines schwefelwasserstoffhaltigen Gasstroms umfassend
(i) Verdichten des schwefelwasserstoffhaltigen Gasstroms in einem Verdichter,
(ii) Spülen des Verdichters mit einer Mischung enthaltend
a) 10 bis 98 Gew.-% Dialkylpolysulfide;
b) 1,9 bis 80 Gew.-% Dialkyldisulfide und
c) 0,1 bis 10 Gew.-% mindestens eines Amins
wobei die Gesamtsumme an Dialkylpolysulfiden, Dialkyldisulfiden und mindestens einem Amin 100 Gew.-% beträgt.

2. Verfahren nach Anspruch 1, **dadurch gekennzeichnet, dass** der Verdichter ein Kreisgasverdichter, bevorzugt ein Flüssigkeitsringverdichter oder ein Schraubenverdichter, ist.

3. Verfahren nach Anspruch 1 oder 2, **dadurch gekennzeichnet, dass** die Verdichtung in Schritt (i) ein- oder zweistufig erfolgt.

4. Verfahren nach einem der Ansprüche 1 bis 3, **dadurch gekennzeichnet, dass** das Spülen in Schritt (ii) kontinuierlich erfolgt.

5. Verfahren nach einem der Ansprüche 1 bis 3, **dadurch gekennzeichnet, dass** das Spülen in Schritt (ii) diskontinuierlich erfolgt.

6. Verfahren nach Anspruch 5, **dadurch gekennzeichnet, dass** das Spülen des Verdichters in Schritt (ii) bei Temperaturen von 30 bis 160°C erfolgt.

7. Verfahren nach einem der Ansprüche 1 bis 6, **dadurch gekennzeichnet, dass** die zum Spülen in Schritt (ii) eingesetzte Mischung
a) 20 bis 95 Gew.-%, besonders bevorzugt 35 bis 90 Gew.-% Dialkylpolysulfide;
b) 4,8 bis 72 Gew.-%, besonders bevorzugt 9,5 bis 60 Gew.-% Dialkyldisulfide;
c) 0,2 bis 8 Gew.-%, besonders bevorzugt 0,5 bis 5 Gew.-% mindestens eines Amins;
wobei die Gesamtsumme an Dialkylpolysulfiden, Dialkyldisulfiden und mindestens einem Amin 100 Gew.-% beträgt,
enthält.

8. Verfahren nach einem der Ansprüche 1 bis 7, **dadurch gekennzeichnet, dass** der schwefelwasserstoffhaltige Gasstrom durch Herstellung von Schwefelwasserstoff aus den Elementen Schwefel und Wasserstoff erhalten wird.

9. Verfahren zur Verdichtung eines schwefelwasserstoffhaltigen Gasstroms gemäß Anspruch 1 umfassend
(i) Verdichten des schwefelwasserstoffhaltigen Gasstroms in einem Verdichter,
(ii) Spülen des Verdichters mit einer Mischung enthaltend
a) 10 bis 98 Gew.-% Dialkylpolysulfide;
b) 1,9 bis 80 Gew.-% Dialkyldisulfide und
c) 0,1 bis 10 Gew.-% mindestens eines Amins,
wobei die Gesamtsumme an Dialkylpolysulfiden, Dialkyldisulfiden und mindestens einem Amin 100 Gew.-% beträgt
(iii) und anschließende Umsetzung des verdichteten schwefelwasserstoffhaltigen Gasstroms zu Alkylmercaptanen, Dialkyldisulfiden und Alkansulfonsäuren.

10. Verfahren nach Anspruch 9, **dadurch gekennzeichnet, dass** Alkylmercaptane hergestellt werden, umfassend die Umsetzung von Alkanolen mit dem verdichteten schwefelwasserstoffhaltigen Gasstrom.

11. Verfahren nach Anspruch 9, **dadurch gekennzeichnet, dass** Dialkyldisulfide hergestellt werden, umfassend
a) Herstellung von Alkylmercaptanen durch Umsetzung von Alkanolen mit dem verdichteten schwefelwasserstoffhaltigen Gasstrom, und
b) Umsetzung der in Schritt a) erhaltenen Alkylmercaptanen durch Oxidation mit Schwefel zu Dialkyldisulfiden.

12. Verfahren nach Anspruch 9, **dadurch gekennzeichnet, dass** Alkansulfonsäuren hergestellt werden, umfassend
a) Herstellung von Alkylmercaptanen durch Umsetzung von Alkanolen mit dem verdichteten schwefelwasserstoffhaltigen Gasstrom,
b) Umsetzung der in Schritt a) hergestellten Alkylmercaptanen durch Oxidation mit Schwefel zu Dialkyldisulfiden; und
c) Oxidation der in Schritt (b) erhaltenen Dialkyldisulfide mit einem Oxidationsmittel zu Alkansulfonsäure.

13. Verwendung einer Mischung enthaltend
a) 10 bis 98 Gew.-% Dialkylpolysulfide;
b) 1,9 bis 80 Gew.-% Dialkyldisulfide und
c) 0,1 bis 10 Gew.-% mindestens eines Amins,
wobei die Gesamtsumme an Dialkylpolysulfiden, Dialkyldisulfiden und mindestens einem Amin 100 Gew.-% beträgt, zur Entfernung von bei der Verdichtung von schwefelwasserstoffhaltigen Gasströmen auftretenden Schwefelablagerungen.

## Claims

1. A process for compressing a hydrogen-sulfide-comprising gas stream, which comprises
(i) compressing the hydrogen-sulfide-comprising gas stream in a compressor,
(ii) flushing the compressor with a mixture comprising
a) 10 to 98% by weight of dialkyl polysulfides;
b) 1.9 to 80% by weight of dialkyl disulfides and
c) 0.1 to 10% by weight of at least one amine,
wherein the sum of dialkyl polysulfides, dialkyl disulfides and at least one amine totals 100% by weight.

2. The process according to claim 1, wherein the compressor is a rotary gas compressor, preferably a liquid ring compressor or a screw compressor.

3. The process according to claim 1 or 2, wherein the compression in step (i) proceeds in one or two stages.

4. The process according to any of claims 1 to 3, wherein the flushing in step (ii) proceeds continuously.

5. The process according to any of claims 1 to 3, wherein the flushing in step (ii) proceeds discontinuously.

6. The process according to claim 5, wherein the flushing of the compressor in step (ii) proceeds at temperatures of 30 to 160°C.

7. The process according to any of claims 1 to 6, wherein the mixture used for flushing in step (ii) comprises
a) 20 to 95% by weight, particularly preferably 35 to 90% by weight, of dialkyl polysulfides;
b) 4.8 to 72% by weight, particularly preferably 9.5 to 60% by weight, of dialkyl disulfides;
c) 0.2 to 8% by weight, particularly preferably 0.5 to 5% by weight, of at least one amine;
wherein the sum of dialkyl polysulfides, dialkyl disulfides and at least one amine totals 100% by weight.

8. The process according to any of claims 1 to 7, wherein the hydrogen-sulfide-comprising gas stream is obtained by producing hydrogen sulfide from the elements sulfur and hydrogen.

9. The process for compressing a hydrogen-sulfide-comprising gas stream according to claim 1, which comprises
(i) compressing the hydrogen-sulfide-comprising gas stream in a compressor,
(ii) flushing the compressor with a mixture comprising
a) 10 to 98% by weight of dialkyl polysulfides;
b) 1.9 to 80% by weight of dialkyl disulfides and
c) 0.1 to 10% by weight of at least one amine,
wherein the sum of dialkyl polysulfides, dialkyl disulfides and at least one amine totals 100% by weight,
(iii) and subsequently reacting the compressed hydrogen-sulfide-comprising gas stream to form alkylmercaptans, dialkyl disulfides and alkanesulfonic acids.

10. The process according to claim 9, wherein alkylmercaptans are produced which comprises reacting alkanols with the compressed hydrogen-sulfide-comprising gas stream.

11. The process according to claim 9, wherein dialkyl disulfides are produced, which comprises
a) producing alkylmercaptans by reacting alkanols with the compressed hydrogen-sulfide-comprising gas stream, and
b) converting the alkylmercaptans obtained in step a) to dialkyl disulfides by oxidation with sulfur.

12. The process according to claim 9, wherein alkanesulfonic acids are produced, which comprises
a) producing alkylmercaptans by reacting alkanols with the compressed hydrogen-sulfide-comprising gas stream,
b) converting the alkylmercaptans produced in step a) to dialkyl disulfides by oxidation with sulfur; and
c) oxidizing the dialkyl disulfides obtained in step b) to alkanesulfonic acid using an oxidizing agent.

13. The use of a mixture comprising
a) 10 to 98% by weight of dialkyl polysulfides;
b) 1.9 to 80% by weight of dialkyl disulfides and
c) 0.1 to 10% by weight of at least one amine,
wherein the sum of dialkyl polysulfides, dialkyl disulfides and at least one amine totals 100% by weight, for removing sulfur deposits which occur in the compression of hydrogen-sulfide-comprising gas streams.

## Revendications

1. Procédé pour la compression d'un flux gazeux contenant du sulfure d'hydrogène, comprenant
(i) la compression du flux gazeux contenant du sulfure d'hydrogène dans un compresseur,
(ii) le rinçage du compresseur avec un mélange contenant
a) 10 à 98% en poids de polysulfures de dialkyle ;
b) 1,9 à 80% en poids de disulfures de dialkyle et
c) 0,1 à 10% en poids d'au moins une amine
la somme totale de polysulfures de dialkyle, de disulfures de dialkyle et d'au moins une amine valant 100% en poids.

2. Procédé selon la revendication 1, **caractérisé en ce que** le compresseur est un compresseur de gaz de recyclage, de préférence un compresseur à anneau liquide ou un compresseur à vis.

3. Procédé selon la revendication 1 ou 2, **caractérisé en ce que** la compression dans l'étape (i) a lieu en un ou deux étages.

4. Procédé selon l'une quelconque des revendications 1 à 3, **caractérisé en ce que** le rinçage dans l'étape (ii) a lieu en continu.

5. Procédé selon l'une quelconque des revendications 1 à 3, **caractérisé en ce que** le rinçage dans l'étape (ii) a lieu en discontinu.

6. Procédé selon la revendication 5, **caractérisé en ce que** le rinçage du compresseur dans l'étape (ii) a lieu à des températures de 30 à 160°C.

7. Procédé selon l'une quelconque des revendications 1 à 6, **caractérisé en ce que** le mélange utilisé pour le rinçage dans l'étape (ii) contient
a) 20 à 95% en poids, de manière particulièrement préférée 35 à 90% en poids, de polysulfures de dialkyle ;
b) 4,8 à 72% en poids, de manière particulièrement préférée 9,5 à 60% en poids de disulfures de dialkyle ;
c) 0,2 à 8% en poids, de manière particulièrement préférée 0,5 à 5% en poids d'au moins une amine ;
la somme totale de polysulfures de dialkyle, de disulfures de dialkyle et d'au moins une amine valant 100% en poids.

8. Procédé selon l'une quelconque des revendications 1 à 7, **caractérisé en ce que** le flux gazeux contenant du sulfure d'hydrogène est obtenu par préparation de sulfure d'hydrogène à partir des éléments soufre et hydrogène.

9. Procédé pour la compression d'un flux gazeux contenant du sulfure d'hydrogène selon la revendication 1, comprenant
(i) la compression du flux gazeux contenant du sulfure d'hydrogène dans un compresseur,
(ii) le rinçage du compresseur avec un mélange contenant
a) 10 à 98% en poids de polysulfures de dialkyle ;
b) 1,9 à 80% en poids de disulfures de dialkyle et
c) 0,1 à 10% en poids d'au moins une amine,
la somme totale de polysulfures de dialkyle, de disulfures de dialkyle et d'au moins une amine valant 100% en poids,
(iii) et la transformation consécutive du flux gazeux comprimé contenant du sulfure d'hydrogène en alkylmercaptans, en disulfures de dialkyle et en acides alcanesulfoniques.

10. Procédé selon la revendication 9, **caractérisé en ce que** des alkylmercaptans sont préparés, comprenant la transformation d'alcanols avec le flux gazeux comprimé contenant du sulfure d'hydrogène.

11. Procédé selon la revendication 9, **caractérisé en ce que** des disulfures de dialkyle sont préparés, comprenant
a) la préparation d'alkylmercaptans par transformation d'alcanols avec le flux gazeux comprimé contenant du sulfure d'hydrogène et
b) la transformation des alkylmercaptans obtenus dans l'étape a) par oxydation avec du soufre en disulfures de dialkyle.

12. Procédé selon la revendication 9, **caractérisé en ce que** des acides alcanesulfoniques sont préparés, comprenant
a) la préparation d'alkylmercaptans par transformation d'alcanols avec le flux gazeux comprimé contenant du sulfure d'hydrogène,
b) la transformation des alkylmercaptans obtenus dans l'étape a) par oxydation avec du soufre en disulfures de dialkyle ; et
c) l'oxydation des disulfures de dialkyle obtenus dans l'étape (b) avec un oxydant en acide alcanesulfonique.

13. Utilisation d'un mélange, contenant
a) 10 à 98% en poids de polysulfures de dialkyle ;
b) 1,9 à 80% en poids de disulfures de dialkyle et
c) 0,1 à 10% en poids d'au moins une amine,
la somme totale de polysulfures de dialkyle, de disulfures de dialkyle et d'au moins une amine valant 100% en poids, pour l'élimination de dépôts de soufre qui surviennent lors de la compression de flux gazeux contenant du sulfure d'hydrogène.
